(19) 

Europäisches
Patentamt
European
Patent Office
Office européen
des brevets

(11) **EP 3 951 398 A1**

(12) **EUROPEAN PATENT APPLICATION**
published in accordance with Art. 153(4) EPC

(43) Date of publication:
**09.02.2022 Bulletin 2022/06**

(21) Application number: **20779436.3**

(22) Date of filing: **10.03.2020**

(51) International Patent Classification (IPC):
***G01N 35/00*** *(2006.01)*

(52) Cooperative Patent Classification (CPC):
**G01N 35/00**

(86) International application number:
**PCT/JP2020/010253**

(87) International publication number:
**WO 2020/195783 (01.10.2020 Gazette 2020/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.03.2019 JP 2019058958**

(71) Applicant: **Hitachi High-Tech Corporation
Minato-ku
Tokyo 105-6409 (JP)**

(72) Inventor: **NAKAJIMA Atsushi
Tokyo 105-6409 (JP)**

(74) Representative: **Strehl Schübel-Hopf & Partner
Maximilianstrasse 54
80538 München (DE)**

(54) **DATA ANALYSIS METHOD, DATA ANALYSIS SYSTEM, AND COMPUTER**

(57) A technique for improving the accuracy of deviation determination based on a regression line of measurement data in an automatic analysis system is provided. A data analysis system according to the present disclosure includes a plurality of first computers that display, on a screen, measurement data obtained by allowing a sample to react with a reagent in one or more automatic analyzers, and a second computer that analyzes the measurement data acquired from each of the first computers and transmits a result of the analysis to the plurality of first computers. The second computer executes a process of acquiring reference data including information on the automatic analyzers, information on the reagent, and the measurement data associated with the information, a process of generating, based on the reference data, integrated regression line information commonly applicable to the plurality of automatic analyzers associated with the information on the automatic analyzers and the information on the reagent, and a process of transmitting the integrated regression line information to the first computers. The first computers execute a process of acquiring the integrated regression line information transmitted from the second computer and displaying the integrated regression line information on a screen

FIG. 3

**Description**

Technical Field

The present invention relates to a data analysis method, a data analysis system, and a computer.

Background Art

An automatic analyzer (hereinafter also referred to as automatic analysis system) for clinical inspection is a device that dispenses a certain amount of a sample and a certain amount of a reagent, allows the sample to react with the reagent by stirring, and analyzes a liquid (reaction liquid) obtained after the reaction. An operator uses the automatic analyzer to measure the absorbance of the reaction liquid for a certain time period and calculates, based on the result of the measurement, the concentration of a component to be analyzed. For example, in analysis for clinical inspection, a device such as an automatic analyzer, a reagent for each analysis item, a standard liquid for calibration of the reagents, a device under analysis, an accuracy control sample to be measured to check states of the reagents, and the like are required. The final analysis performance can be obtained by combining other factors besides these factors. Examples of the other factors are the accuracy of an amount dispensed by the device, the uniformity of the insides of bottles of the reagents, stability at the time of storage, the degree of chemical reaction, in particular, the efficiency of stirring the reagents and samples, degrees of cleaning of reaction vessels, and the stability of the standard liquid. As described above, there are multiple factors that govern the analysis performance. In addition, factors (factors that directly affect the analysis performance) that affect the analysis performance and are included in the device are configurations such as a sampling mechanism, a reagent dispensing mechanism, a stirring mechanism, an optical system, a reaction vessel, and a constant-temperature tank. In addition, as factors that affect the analysis performance and are not included in the device, there are a reagent, a sample, the liquidity of a control specimen, and the like. The analysis performance is affected by various factors as described above. Therefore, when the automatic analyzer is used, it is necessary to confirm these factors (affecting factors) and confirm whether clinical inspection can be normally performed.

A calibration process is performed using a standard liquid for each reagent bottle for each item in the automatic analyzer. Specifically, a blank liquid and a standard liquid are measured to determine the origin, absorbance per unit concentration is calculated, and a conversion factor (hereinafter abbreviated as K factor) is calculated. Generally, a clinical laboratory technologist confirms the magnitude of the absorbance and a change in the K factor over time and determines the quality of the result of the calibration.

In addition, after the calibration, an accuracy control sample of known concentration is measured and a difference from a standard value is confirmed (accuracy control). Normally, during the time when a patient's specimen is measured, an accuracy control sample is periodically measured for a certain time period and a deviation from an allowable value is confirmed. When it exceeds the allowable value, it is considered that there is a problem with either a reagent or the device, and inspection is performed. In addition, data to be used in daily inspection is confirmed using reaction process data. A method therefor varies depending on an analysis method. Measurement methods in clinical inspection are classified into two types, a rate method and an end point method. Here, the end point method is briefly described.

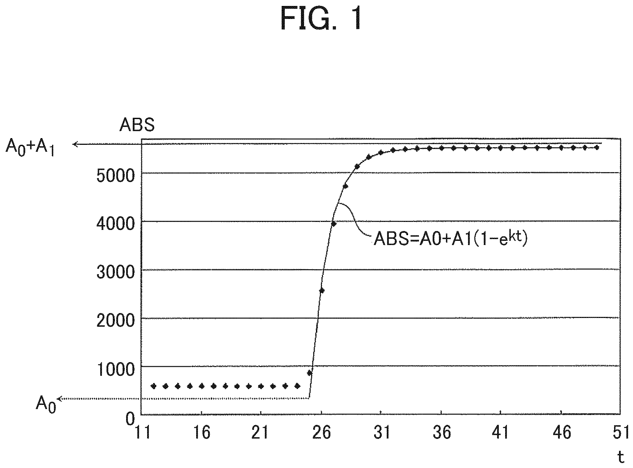

The end point method relates to a method for measuring components that are mainly protein, fat, and the like contained in a sample. When a component contained in a sample reacts with a reagent, and binding reaction is quick, the reaction ends within a short time period and the concentration of a reaction product is a certain value. When the reaction time period is long, it takes time for the concentration of the reaction product to reach the certain concentration. FIG. 1 is a schematic diagram illustrating the relationship between time and the reaction product. In the end point method (the same applies to the rate method) that is a method for accurately calculating the concentration of a measurement target object in biochemistry analysis, the relationship between absorbance in chromogenic reaction thereof and time is approximated by the least-squares method of $y = A + (B - A) / e^{Kt}$ and the concentration of the measurement target object is calculated. As a conventional method for detecting abnormal data at the time of measurement in the end point method, there is prozone check. In addition, for example, when a reagent that is obtained using immunoturbidimetry and is IgA (immunoglobulin A), CRP (C-reactive protein), or the like is used, protein may be precipitated as a deposit due to salt concentration of reagent composition. A reaction process may be unstable due to the deposit and the instability actually occurs in second half of the reaction time in many cases. When the instability occurs at a photometry point section used for concentration calculation, it is not possible to accurately obtain a measured value. As methods for checking this, there are an antibody re-addition method, a reaction rate ratio method, and the like. Each of the methods is a method in which, when a measured value exceeds a limit value specified in a parameter, an alarm is output.

Citation List

Patent Literature

Patent Literature 1: Japanese Patent Application Laid-Open No. 2008-047033
Patent Literature 2: Japanese Patent Application Laid-Open No. 2014-247715

Summary of Invention

Technical Problem

In recent years, with improvement in performance of automatic analyzers, it is possible to analyze various items with high accuracy even when a small amount of a sample and a small amount of a reagent are used. On the other hand, analysis cannot be accurately performed due to a small abnormality in each section of a device, a small change in the quality of a specimen or a reagent, or the like in some cases. An automatic analyzer for clinical inspection measures, at certain time intervals, the absorbance of a solution in which a sample has reacted with a reagent, and measures the rate of change in the absorbance and the final absorbance based on the absorbance measured over time. From these data, the concentration of a measurement target substance and an enzyme activity value are calculated. During monitoring of a reaction process, the automatic analyzer performs sampling and dispenses and stirs a reagent. These processes include a plurality of factors of errors (for example, an error caused by a difference between degrees of stirring, an error caused by the amount of air bubbles that occur at the time of sampling, and the like). In particular, since whether stirring is performed and the stirring level cannot be quantitatively evaluated and there is not a determination standard in conventional techniques, reproductivity was not stable, measured values were not continuous, and evaluation such as the presence or absence of measured values that clearly had some kind of defect was unclear. It is necessary to detect an abnormality for factors that directly affect reaction in the case where a reagent is diluted due to cleaning water for a reagent probe, the case where a user erroneously mixes another solution into a reagent, or another case, and it is necessary to cause an automatic analyzer to prompt the user to perform reinspection or maintain the device.

It is difficult for a laboratory technologist (operator) who is a user of the automatic analyzer to visually check an entire reaction process in daily inspection work. In particular, when a measured value is in a normal value range, the user tends to overlook a reaction abnormality and may give an inaccurate result.

To solve the problems, Patent Literature 1 proposes a technique for approximating reaction process data obtained by measuring the relationship between absorbance in reaction and time by an automatic analyzer and using the data as a parameter to evaluate the reactivity. Specifically, a technique for approximating the reaction process data obtained by measuring the relationship between the absorbance in the reaction and time by the automatic analyzer to $ABS = A0 + A1(1 - e^{-kt})$ using the least-squares method, where A0 is the absorbance at the start time of the reaction, A1 is the final absorbance in the reaction, and k is a reaction speed constant, and using, as a residual error, the sum of differences between approximated values obtained as a result of the approximation and measured values.

In addition, Patent Literature 2 obtains the effect of improving the accuracy of the accuracy control of a reaction process and easily confirming a reason for a deviation by a user by performing a process of determining a deviation of data using a reference data distribution obtained by accumulating, as evaluation parameters, the absorbance A0 at the start time of the reaction, the final absorbance A1 in the reaction, the reaction speed constant k, and the residual error described in Patent Literature 1, calculating, from the reference data distribution, a reaction process curved line ideal for the deviating data, and displaying a reaction process curved line of the deviating data and the ideal reaction process curved line simultaneously. In each of the techniques disclosed in Patent Literature 1 and Patent Literature 2, a parameter to evaluate a deviation for each measurement item and each reaction lot for one automatic analyzer is calculated, and a reference data distribution diagram and a regression line that depend on reference data measured by an automatic analyzer are generated.

However, the reference data distribution and the regression line depend on a specimen. Therefore, when a result of measuring the specimen is biased, the regression line is specialized for the automatic analyzer unit. It is considered that the accuracy of determining a deviation using a regression line for one automatic analyzer may not be sufficient to objectively determine a deviation in a reaction process for each reagent used by a user and each reagent lot due to a device difference specific to the device, the bias of the specimen, and the like.

The present disclosure has been made in view of the foregoing situations and provides a technique for improving the accuracy of determining a deviation of measurement data from a regression line in an automatic analysis system.

Solution to Problem

To solve the foregoing problem, a data analysis method according to the present disclosure includes causing each of a plurality of first computers to acquire measurement data obtained by allowing a sample to react with a reagent in automatic analyzers, causing a second computer connected to the plurality of first computers to analyze the measurement data acquired from each of the first computers and generate a result of the analysis, causing the second computer to transmit the analysis result to the plurality of first computers, and causing the plurality of first computers to display the analysis result acquired from the second computer on display screens of the first computers. In the generating of the analysis result, the second computer executes a process of acquiring reference data including information on the automatic analyzers, information on the reagent, and the measurement data associated with the information, a process of generating integrated deviation determination line information commonly applicable to the plurality of automatic analyzers associated with the information on the automatic analyzers and the information on the reagent, and a process of transmitting the integrated regression line information to the first computers. In the displaying on the display screens, the first computers execute a process of acquiring the integrated regression line information transmitted from the second computer and displaying the integrated regression line information on the display screens.

Other characteristics relating to the present disclosure will be clarified from the description of the present specification and the accompanying drawings. In addition, aspects of the present disclosure are achieved by elements, combinations of various elements, the following detailed description, and the appended claims.

It should be understood that the description of the present specification is merely an exemplary example and is not intended to limit the claims or examples of application in any way.

Advantageous Effects of Invention

According to the present disclosure, it is possible to improve the accuracy of determining a deviation of measurement data from a regression line in an automatic analysis system.

Brief Description of Drawings

Fig. 1 is a diagram illustrating an outline of a logistic curved line.

Fig. 2 is a diagram illustrating an example of a principle overall configuration of an automatic analysis system according to an embodiment.

Fig. 3 is a diagram illustrating an example of a configuration of an automatic-analysis data analysis system according to the embodiment.

Fig. 4 is a diagram illustrating an example of an internal block configuration of an integrated analysis server 309 according to the embodiment.

Fig. 5 is a diagram illustrating an example of an internal block configuration of each of remote terminals 303 and 310 according to the embodiment.

Fig. 6 is a diagram illustrating an example of an internal block configuration of each of automatic analysis systems 302 to 307 according to the embodiment and the like.

Fig. 7 is a diagram illustrating an example of a configuration of a reference data information table 701 (hereinafter also referred to as reference data 701) storing reference data from the automatic analysis systems.

Fig. 8 is a diagram illustrating a data configuration 801 (example) of the integrated analysis server that is constituted by the reference data information table accumulated in the integrated analysis server 309.

Fig. 9 is a diagram illustrating an example of a configuration of a deviation determination screen 900 including an integrated regression line and a deviation determination line displayed in each automatic analysis system or the remote terminal 303.

Fig. 10 is a flowchart describing a process that is executed by the integrated analysis server 309 according to the embodiment.

Fig. 11 is a flowchart describing details of step 1005 (integrated regression line calculation process) illustrated in Fig. 10.

Description of Embodiments

The present embodiment discloses a function of monitoring reaction at the time of clinical inspection analysis, and the function relates to an automatic analysis system that qualitatively or quantitively analyzes blood, urine, and other biological samples. The function improves the accuracy of a reaction process approximation method.

Hereinafter, the present embodiment is described with reference to the accompanying drawings. In the accompanying drawings, components that are functionally the same are represented by the same reference signs in some cases. The accompanying drawings illustrate the specific embodiment and implementation examples based on the principles of the present disclosure, and these are provided to understand the present disclosure and are not used to interpret the present disclosure in a limited manner.

Although the present embodiment is described in sufficient detail for those skilled in the art to implement the embodiment, it is necessary to understand that other implementation and embodiments are possible and changes in configurations and structures and various replacements of components can be made without departing from the scope and spirit of the technical idea of the present disclosure. Therefore, the following description should not be interpreted to be limited thereto.

Furthermore, as described later, the present embodiment may be implemented by software executed in a general-purpose computer or may be implemented by dedicated hardware or a combination of software and hardware.

Although each information item disclosed herein is in a "table" format in the following description, each of these information items may not be represented in a table data structure and may be represented in a data structure such as a list, a DB, or a queue or in another data structure. Therefore, a "table", a "list", a "DB", a "queue", and the like are merely referred to as "information" in order to indicate that those do not depend on a data structure in some cases.

"No", "identification information", an "identifier", a "name", a "name", or an "ID" can be used to explain a detail of each information item, and these items can be replaced with each other.

<Entire Configuration of Automatic Analysis System (Automatic Analyzer)>

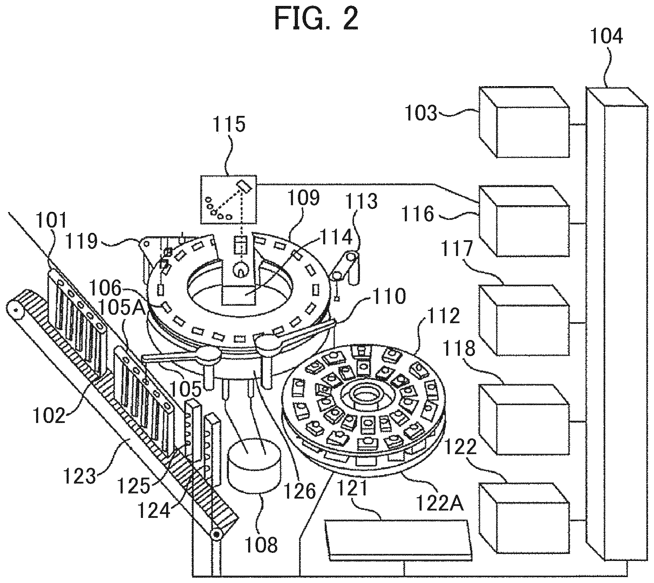

Fig. 2 is a diagram illustrating an example of a principle overall configuration of the automatic analysis system according to the present embodiment. 109 indicates a reaction disk, and a plurality of reaction vessels 106 are set on the concentric circumference of the reaction disk. The reaction disk 109 is rotatably attached and has a rotation driving mechanism (not illustrated). The reaction disk 109 is maintained at a predetermined temperature by a thermal insulation tank 126 coupled to a constant-temperature tank 108.

112A indicates a reagent disk, and a plurality of reagent bottles 112 storing various reagents are set on the concentric circumference of the reagent disk. A sample dispensing mechanism 105, a reagent dispensing mechanism 110, a stirring device 113, a cleaning device 119, a light source 114, and a multiwavelength photometer 115 are arranged around the reaction disk 109 and the reagent disk 112A.

A rack transport device 123 is arranged on the rotation circumference of the sample dispensing mechanism 105 and along a normal direction of the reaction disk 109. In addition, a rack number reading device 124 and a specimen ID reading device 125 are arranged along a transport line. Mechanical operations of these devices are all controlled by a computer 103 via an interface 104.

A plurality of sample vessels 101 storing a sample are arranged in a specimen rack 102. The specimen rack 102 is transported on the transport line by the rack transport device 123. A sequential number is assigned to each specimen rack 102 and read by the rack number reading device 124 in the middle of the transport on the transport line.

After that, when an ID number is assigned to each sample vessel held in the specimen rack 102, an ID number of a specimen is read by the specimen ID reading device 125. After that, the specimen rack 102 is moved to a position where the first sample vessel 101 is positioned directly under a sample dispensing probe 105A of the sample dispensing mechanism 105. Information read by the rack number reading device 124 and the specimen ID reading device 125 is all transmitted to the computer 103 via the interface 104.

The sample dispensing mechanism 105 uses the sample dispensing probe 105A to dispense, into a reaction vessel 106, a predetermined amount of a sample stored in the sample vessel 101 under control by the computer 103. When the dispensing is ended for one sample vessel 101, the specimen rack 102 is moved so that the next sample vessel 101 is positioned directly under the sample dispensing probe 105A.

The reaction vessel 106 storing the dispensed sample is rotated and moved on the reaction disk 109 by a rotational operation of the reaction disk 109. During this, for the sample in the reaction vessel 106, a reagent in the reagent bottle 112 is dispensed by the reagent dispensing mechanism 110, a reaction liquid is stirred by the stirring device 113, and absorbance is measured by the light source 114 and the multiwavelength photometer 115. After that, the reaction vessel 106 for which the analysis has been completed is cleaned by the cleaning device 119. A signal of the measured absorbance is input to the computer 103 via an A/D converter 116 and the interface 104 and converted into concentration of a measurement target component within the sample. Data of the converted concentration is displayed on a CRT 118 via the interface 104 or printed by a printer 117 and stored in a storage device 122.

The sample dispensing mechanism 105, the reaction disk 109, the rack transport device 123, the reagent dispensing mechanism 110, the stirring device 113, and the cleaning device 119 are mechanisms that are driven by a pulse motor not illustrated. In addition, although not illustrated, a plurality of automatic analyzers can be coupled to each other to operate as one automatic analysis system.

<Example of Configuration of Automatic-Analysis Data Analysis System>

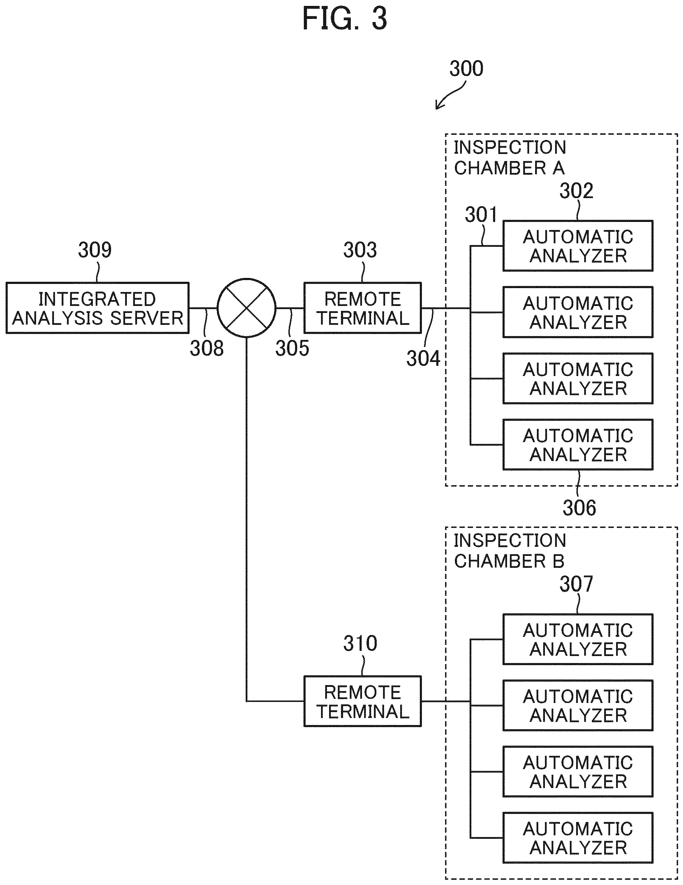

Fig. 3 is a diagram illustrating an example of a configuration of an automatic-analysis data analysis system 300. The automatic-analysis data analysis system 300 includes a plurality of automatic analysis systems 302 within an inspection chamber A, a plurality of automatic analysis systems 302 within an inspection chamber B, a remote terminal 303 connected to the automatic analysis systems 302 within the inspection chamber A via a communication line (LAN) 301, a remote terminal 310 connected to the automatic analysis systems 302 within the inspection chamber B via a communication line (LAN), and an integrated analysis server 309 connected to the remote terminals 303 and 310 via communication lines (dedicated line, Internet line, or the like) 305 and 308. Fig. 3 illustrates the number of automatic analysis systems illustrated and the number of remote terminals illustrated, but it is preferable that at least one automatic analysis system be present in each of the inspection chambers and it is preferable that two or more remote terminals be present.

Each of the automatic analysis systems 302 transmits reaction process data to the remote terminal 303. The remote terminal 303 generates reference data 701 for each of the automatic analysis systems based on the reaction process data. The remote terminal 303 transmits the generated reference data to the integrated analysis server 309 via the communication lines 305 and 308.

The integrated analysis server 309 accumulates, as reference data (data configuration 801 unit), data of an item code, a device lot, a reagent lot, and the like. The integrated analysis server 309 uses the accumulated reference data to calculate integrated regression line information and integrated deviation determination line information 904 and transmits the integrated regression line information and the integrated deviation determination line information 904 to the remote terminal 303 and/or the remote terminal 310 via the communication lines 305 and 308.

The remote terminal 303 displays a distribution diagram of the reference data of the automatic analysis systems 302 and/or 306 installed in each of the inspection chambers, a distribution diagram of measurement data, an integrated regression line 903 generated from the regression line information, and the integrated deviation determination standard line 904.

Each of the integrated analysis server 309 and the remote terminal 303 is constituted by a computer and includes, for example, a memory or a storage device that stores various programs, various parameters, and various data, a processor that executes the various programs, an input device that inputs an instruction and data from an operator, an output device (for example a monitor, a printer, or the like) that outputs (for example, displays, prints, or the like) calculation results and the like, and a communication device.

In the system, a computer 103 of an automatic analyzer or the processor 3031 of the remote terminal described later can be referred to as first computer, and the processor 3091 of the integrated analysis server described later can be referred to as second computer. The remote terminals and the integrated analysis server are not limited based on the names, and it is sufficient if the remote terminals and the integrated analysis server are computers having predetermined functions. Therefore, a process of the first computer can be executed by the computer 103 of the automatic analyzer, and a process of the second computer can be executed by the processor 3031 of the one remote terminal.

<Example of Internal Configuration of Integrated Analysis Server>

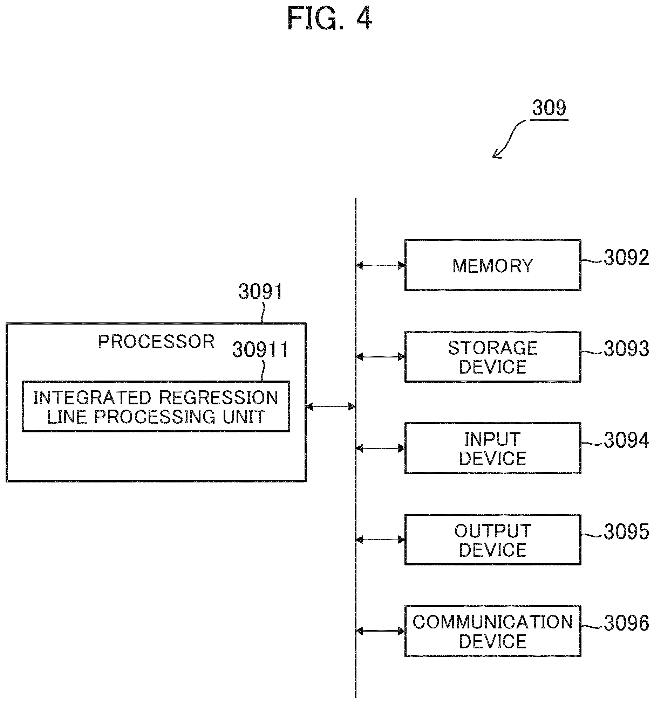

Fig. 4 is a diagram illustrating an example of an internal block configuration of the integrated analysis server 309 according to the present embodiment. The integrated analysis server 309 can be enabled by a computer. For example, the integrated analysis server 309 includes the processor 3091 that executes various installed programs, a memory 3092 storing the various programs and various parameters, a storage device 3093 for storing calculation results and acquired information, an input device 3094 that is constituted by a keyboard, a touch panel, various buttons, a microphone, and the like (or may be one device among these input devices or may include a plurality of types of input devices among these input devices), an output device 3095 that is constituted by a display device, a printer, a speaker, and the like (or may be one device among these output devices or may include a plurality of types of output devices among these output devices), and a communication device 3096 that is connected to the communication line (network) 308 and communicates with the plurality of remote terminals 303, 310, ⋯ (and may directly communicate with the plurality of automatic analysis systems 302, 306, and 307, ⋯). As described above, the integrated analysis server is the second computer.

The processor 3091 reads the various programs (including an integrated regression line process program) from the memory 3092, loads the programs into an internal memory (not illustrated), and appropriately executes the programs. Fig. 4 illustrates a state in which the integrated regression line process program is loaded in the internal memory of the processor 3091. The processor 3091 executes at least an integrated regression line processing unit 30911 as a program. Process details of the integrated regression line processing unit 30911 are described later.

<Example of Internal Configuration of Each Remote Terminal)

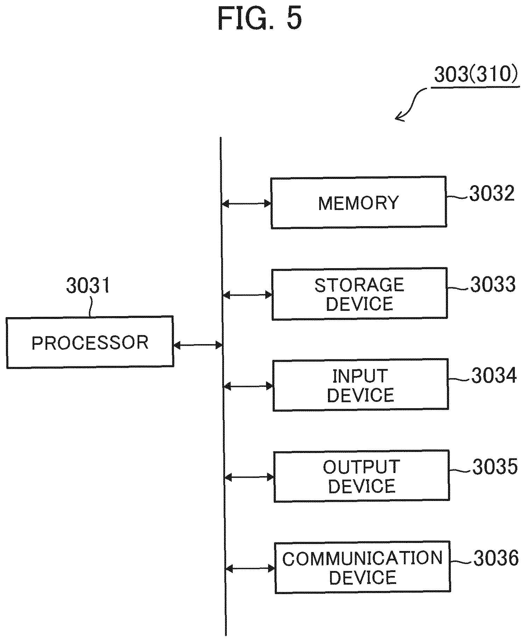

Fig. 5 is a diagram illustrating an example of an internal block configuration of each of the remote terminals 303 and 310 according to the present embodiment.

The remote terminal 303 can be enabled by a computer and includes, for example, the processor 3031 that executes installed various programs, a memory 3032 storing the various programs and various parameters, a storage device 3033 for storing calculation results and acquired information, an input device 3024 that is constituted by a keyboard, a touch panel, various buttons, a microphone, and the like (or may be one device among these input devices or may include a plurality of types of input devices among these input devices), an output device 3035 that is constituted by a display device, a printer, a speaker, and the like (or may be one device among these output devices or may include a plurality of types of output devices among these output devices), and a communication device 3036 that is connected to the communication lines (networks) 304 and 305 and communicates with the integrated analysis server 309 and the plurality of automatic analysis systems 302 to 306 installed in the same inspection chamber (for example, the inspection chamber A) .

As described above, for example, the remote terminal 303 displays the integrated regression line 903 generated from the regression line information and the integrated deviation determination standard line 904 in the reference data distribution diagram and the measurement data distribution diagram of the automatic analysis systems 302 and 307 installed in each of the inspection chambers.

The remote terminals 303 to 310, ··· may enable the automatic-analysis data analysis system 300 using the integrated analysis server 309 and the plurality of automatic analyzers 302 to 307 without using constituent components required to enable the automatic-analysis data analysis system 300. In addition, the one remote terminal 303 can execute the process of the second computer of the integrated analysis server 309.

<Example of Internal Configuration of Automatic Analysis System>

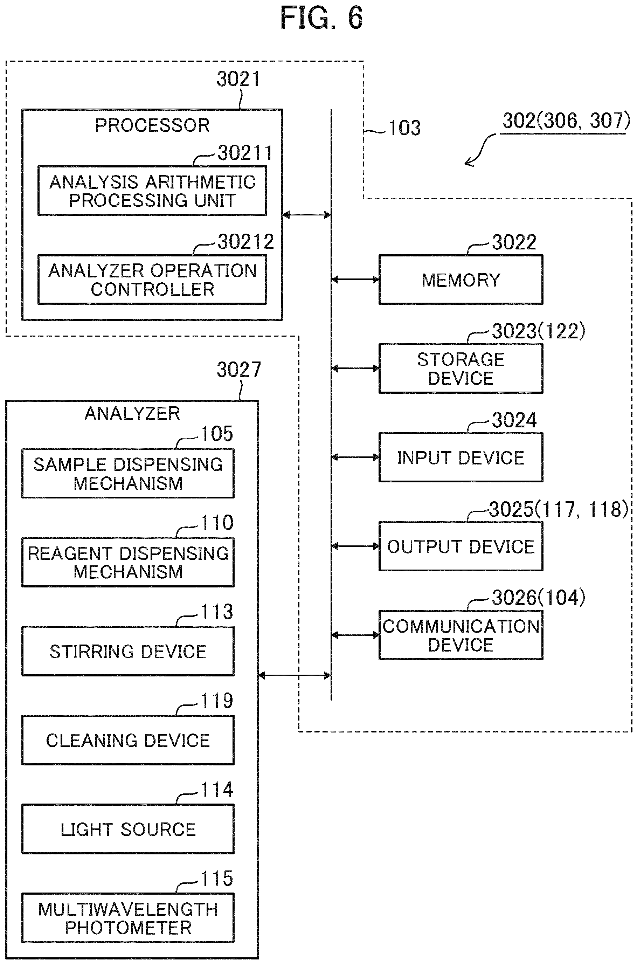

Fig. 6 is a diagram illustrating an example of an internal block configuration of each of the automatic analysis systems 302 to 307 according to the present embodiment and the like. The automatic analysis systems 302 to 307 and the like can be enabled by the computer 103 and an analyzer (automatic analyzer: an automatic analyzer constituted by a combination of the computer 103 and the automatic analyzer 3027) 3027. For example, the internal configuration of the computer 103 includes a processor 3021 that executes various installed programs, a memory 3022 storing the various programs and various parameters, a storage device 3023 (corresponding to the foregoing storage device 122) for storing calculation results and acquired information, an input device 3024 that is constituted by a keyboard, a touch panel, various buttons, a microphone, and the like (or may be one device among these input devices or may include a plurality of types of input devices among these input devices), an output device 3025 that is constituted by a display device, a printer, a speaker, and the like (or may be one device among these output devices or may include a plurality of types of output devices among these output devices and corresponds to the foregoing printer 117 or the CRT 118), and a communication device 3026 that is connected to the communication line (network) 305 and communicates with the plurality of automatic analysis systems 302 to 306 (and may directly communicate with the integrated analysis server 309) provided for the corresponding remote terminal 303. In addition, as described with reference to Fig. 1, the analyzer 3027 includes the sample dispensing mechanism 105, the sample dispensing mechanism 110, the stirring device 113, the cleaning device 119, the light source 114, and the multiwavelength photometer 115.

The processor 3021 reads various programs (including an analysis arithmetic processing program and an analyzer operation control program) from the memory 3022, loads the programs into an internal memory (not illustrated), and executes the programs. Fig. 6 illustrates a state in which the analysis arithmetic processing program and the analyzer operation control program are loaded in the internal memory of the processor 3021. An analysis arithmetic processing unit 30211 executes a process of analyzing data acquired by the analyzer 3027, for example. In addition, an analyzer operation controller 30212 controls the analyzer 3027 to cause the analyzer 3027 to operate in accordance with an instruction input from the input device 3024, for example.

The computer 103 and the analyzer (automatic analyzer) 3027 may be separated from each other and installed at different positions to constitute an automatic analysis system. In addition, a plurality of analyzers (automatic analyzers) 3027 may be connected to the computer 103 to constitute an automatic analysis system. Furthermore, the computer 103 of the automatic analysis system can be used as the first computer.

<Reference Data Information Table>

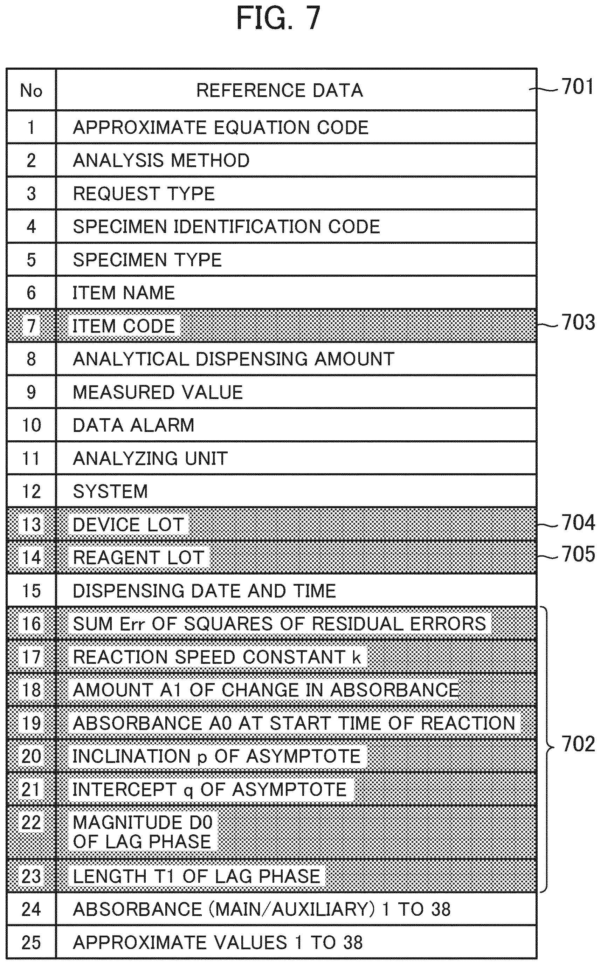

Fig. 7 is a diagram illustrating an example of a configuration of a reference data information table 701 (hereinafter referred to as reference data 701 in some cases) storing reference data from the automatic analysis systems.
As illustrated in Fig. 7, the reference data table 701 is generated by storing reaction process data (including measurement data) transmitted from the automatic analysis systems 302 and 307.

The reference data 701 includes, as configuration information, an item code 703 assigned to a reagent, a device type name of each automatic analyzer in the automatic analysis system, device information (hereinafter represented as device lot) 704 indicating a production unit, a reagent lot 705 indicating a production unit of the reagent, and an evaluation parameter 702 (refer to Patent Literature 2 for details of the evaluation parameter). Regarding the item code 703, when a plurality of pharmaceutical manufacturers are present for each reagent, reagents of the same type that are produced by the companies may be different and thus different codes are assigned to the reagents.

<Example of Data Configuration in Integrated Analysis Server>

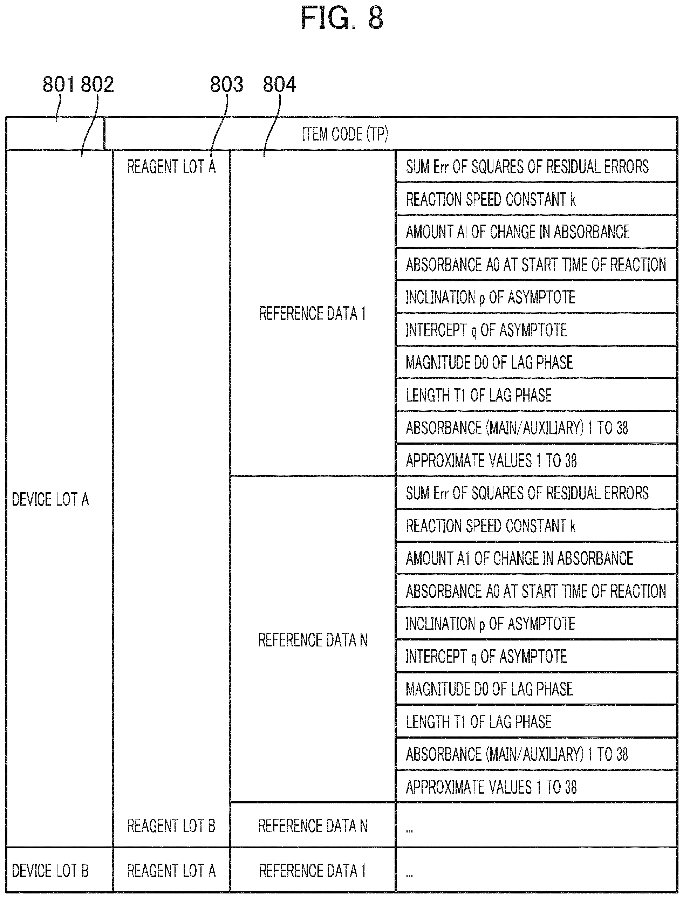

Fig. 8 is a diagram illustrating a data configuration 801 (example) of the integrated analysis server that is constituted by the reference data information table accumulated in the integrated analysis server 309.

The integrated analysis server 309 uses (holds) the data configuration 801 to manage each reference data item 804 corresponding to each reagent lot 803 in units of device lots 802.

In Fig. 8, layers of the device lot 802 and the reagent lot 803 are configured for each item code 703 of the reference data 701, but the data configuration 801 is not limited to this configuration as long as mutual links are set.

<Example of Configuration of Deviation Determination Screen>

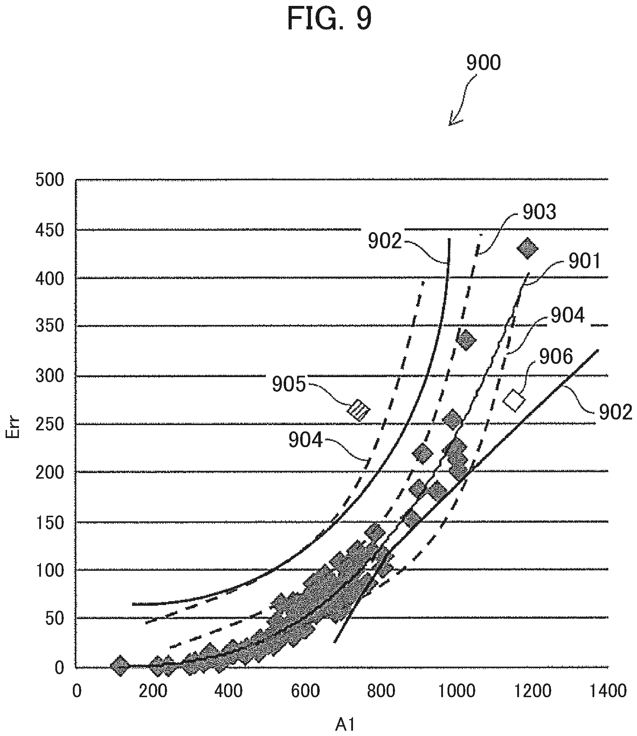

Fig. 9 is a diagram illustrating an example of a configuration of a deviation determination screen 900 including an integrated regression line and a deviation determination line that are displayed in each automatic analysis system or the remote terminal 303.

On the deviation determination screen 900 illustrated in Fig. 9, a regression line 901 of a single automatic analyzer and a deviation determination line 902 of a single automatic analysis system are displayed in a distribution diagram of reference data and a distribution diagram of measurement data. In addition, on the screen 900, the deviation determination line 902 of the single automatic analysis system is displayed as a threshold to distinguish deviating reaction process data and normal reaction process data. Furthermore, on the deviation determination screen 900, the integrated regression line 903 and the integrated deviation determination line 904 are displayed while overlapping each other and are used as standard thresholds for reagents and reagent lots to distinguish between deviations and normal values.

From the deviation determination screen 900 having the foregoing configuration, it is found that reaction process data 905 that deviates from the deviation determination line 902 of the single automatic analysis system and the integrated deviation determination line 904 needs to be reinspected. In addition, it is found that a user needs to check details of reaction process data 906 deviating from the integrated deviation determination line 904 and not deviating from the deviation determination line 902 of the single automatic analysis system, and to determine whether the reaction process data 906 is to be reinspected.

There is a possibility that details of the reaction process data 906 that need to be checked may be overlooked when only a conventional deviation determination line 902 of a single automatic analysis system is used. However, when the deviation determination screen 900 having the foregoing configuration is used, a user can extract data of which details need to be checked by displaying the integrated deviation determination line 904, and the accuracy of deviation determination is improved. This display is not limited to the display screen of each automatic analysis system or the display screen of the remote terminal and may be a display screen of a computer, a terminal, or the like relating to each automatic analysis system or the remote terminal.

<Details of Process by Integrated Analysis Server>

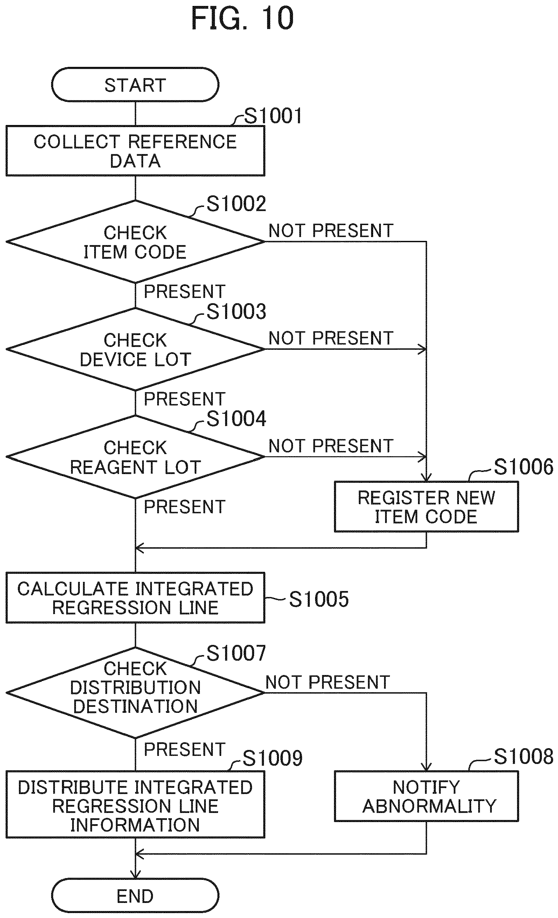

Fig. 10 is a flowchart describing a process that is executed by the integrated analysis server 309 according to the present embodiment. This process may be interpreted as a process that is executed in the second computer. The process represented in the flowchart of Fig. 10 is enabled by a program, for example. In this case, the processor 3091 of the integrated analysis server 309 reads the program from the memory 3092 or the storage device 3093 and executes the program, thereby enabling the integrated regression line processing unit 30911. In the following description, it is assumed that a process of each step illustrated in Fig. 10 is enabled by the processor 3091 (or the integrated regression line processing unit 30911 may be a main operational unit).

(i) Step 1001

When the integrated analysis server 309 periodically (for example, once a day) communicates with the remote terminals 303 and 310 (a trigger for connection of the remote terminals and the integrated analysis server may be from the remote terminals or from the integrated analysis server), the integrated analysis server 309 activates an integrated regression line calculation process. Then, the processor (hereinafter referred to as processor) 3091 (integrated regression line processing unit 30911) of the integrated analysis server 309 collects reference data from the remote terminals 303 and 310. Specifically, in the integrated analysis server 309, the processor 3091 acquires, from the connected remote terminals 303 and 310, the reference data 401, device information (information of a device lot in this case) 404 of the connected automatic analysis systems, and a reagent lot 405 of a reagent used.

(ii) Step 1002

The processor 3091 checks an item code included in the reference data. Specifically, the processor checks whether the item code 403 of the acquired reference data is already registered in the data configuration 801 of the integrated analysis server 309. When the item code 403 is already registered in the data configuration 801 of the integrated analysis server 309 (present in step 1002), the process proceeds to step 1003. When the item code 403 is not registered in the data configuration 801 of the integrated analysis server 309 (not present in step 1002), the process proceeds to step 1006.

(iii) Step 1003

The processor 3091 checks whether the device lot is already registered in the data configuration 801 of the integrated analysis server 309 for the already registered item code. When the device lot is an already registered device lot 502 (present in step 1003), the process proceeds to step 1004. When the acquired device lot is not registered in the data configuration 801 of the integrated analysis server 309 (not present in step 1002), the process proceeds to step 1006.

(iv) Step 1004

The processor 3091 checks whether a reagent lot is already registered in the data configuration 801 of the integrated analysis server 309 for the registered device lot 802. When the reagent lot is an already registered reagent lot 702 (present in step 1004), the process proceeds to step 1005. When the acquired regent lot is not registered in the data configuration 801 of the integrated analysis server 309 (not present in step 1004), the process proceeds to step 1006.

(v) Step 1005

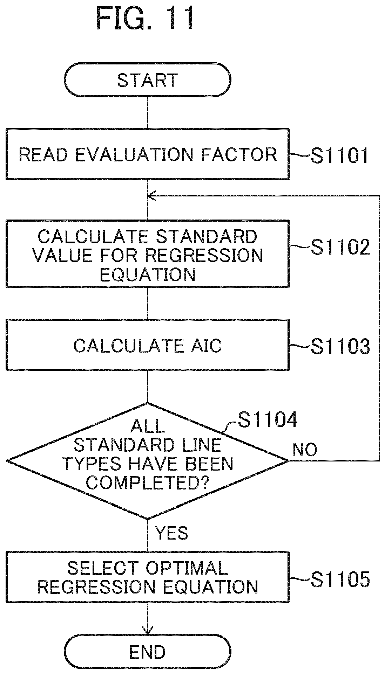

The processor 3091 adds the acquired reference data 701 to the reference data 804 of the data configuration 801 of the integrated analysis server to generate an integrated regression line. Specifically, the processor generates an integrated regression line and an integrated deviation determination line using reference data N from reference data 1 in units of the item code 703 (Fig. 7), the device lot 802, and the reagent lot 803 (refer to Fig. 8). A process of calculating the integrated regression line and the integrated deviation determination line is described later in detail using Fig. 11.

(vi) Step 1006

The processor 3091 registers (newly adds), as a new item code, information that is not registered in the data configuration 801 of the integrated analysis server and indicates the item code 703, the device lot 704, or the reagent lot 705 to the data configuration 801.

(vii) Step 1007

The processor 3091 stores, to the storage device 3093 or the like, the integrated regression line information and the integrated deviation determination line information calculated in step 1006, and checks whether a remote terminal of a distribution destination is present. Whether the remote terminal of the distribution destination is present is determined, for example, by checking whether the device lot 802 and the reagent lot 803 that are the original data for the generation of the integrated regression line information are already registered in the remote terminals 303 and 310 connected to the integrated analysis server 309.
When the processor 3091 determines that the remote terminal of the distribution destination is not present ("not present" in step 1007), the process proceeds to step 1008. When the processor 3091 determines that the remote terminal of the distribution destination is present ("present" in step 1007), the process proceeds to step 1009. The case where the device lot 802 and the reagent lot 803 that are information for the generation of the integrated regression line information are already registered in the data configuration 801 but the remote terminal of the distribution destination is not present is, for example, the case where a reagent corresponding to a reagent lot registered in the data configuration 801 was previously used in an automatic analysis system of the device lot but is not currently used, the case where an automatic analysis system associated with the foregoing device lot 802 is not present among automatic analysis systems currently managed by the remote terminal, or the like. This is a measure to handle the situation where a reagent or the like that was used before is not currently used.

(viii) Step 1008

The processor 3091 displays, for example, an abnormality (absence of the remote terminal of the distribution destination) on a screen of the display device 3095 of the integrated analysis server 309 and ends the process illustrated in Fig. 10.

(ix) Step 1009

The processor 3091 distributes the generated integrated regression line information and the generated integrated deviation determination line information to either one or both of the remote terminals 303 and 310 of the distribution destination and ends the process illustrated in Fig. 10. The remote terminal 303 or 310 that has received the integrated regression line information uses a regression line function to execute an integrated regression line drawing process and an integrated deviation determination line drawing process based on the integrated regression line information. The regression line function is calculated using a function that is the same as or similar to a regression line for one automatic analysis system. Specifically, each of the remote terminals 303 and 310 generates a regression line 901 and a deviation determination line 902 for a single automatic analysis system 302 or 307 managed by the remote terminal and displays, on the deviation determination screen, the lines 901 and 902, and the integrated regression line 903 and the integrated deviation determination line 904 received from the integrated analysis server 309. The process that is described with reference to Fig. 10 and executed by the remote terminal may be executed by the computer 103 of the automatic analysis system, and the process of the integrated analysis server 309 may be executed by the one remote terminal 303. By the foregoing process, regression lines obtained from all facilities are used as standard indices to improve the accuracy of deviation determination by a user.

<Details of Integrated Regression Line Calculation Process>

Fig. 11 is a flowchart describing details of step 1005 (integrated regression line calculation process) of Fig. 10. To execute the process (integrated regression line calculation process) on the regression line function, a sufficient amount of reference data is collected (step 1001). When the accumulation is completed, the process is executed.

(i) Step 1101

The processor 3091 (integrated regression line processing unit 30911) acquires the evaluation parameter 702 for reference data that has been accumulated, as an evaluation factor reading operation.

(ii) Step 1102

The processor 3091 executes a regression equation standard value calculation process and represents a distribution of a combination of evaluation parameters using a regression equation. As the regression equation representing the distribution of the evaluation factors, for example, five types, a 0th order function, a linear function, a quadratic function, a logarithmic function, and an exponential function are used. The functions are the following regression equations.

The 0th order function: $Y = C_1$
The linear function: $Y = C_1 X + C_2$
The quadratic function: $Y = C_1 X^2 + C_2 X + C_3$
The logarithmic function: $Y = C_1 \log(C_3 X + C_4) + C_2$
The exponential function: $Y = C_1 \exp(C_3 X + C_4) + C_2$
Where X and Y are a combination of evaluation parameters, and $C_k$ (k = 1 to 4) indicates a function for a regression equation.

(iii) Step 1103

The processor 3091 executes AIC calculation. The AIC calculation is a process of calculating index data to be used to select the optimal regression equation among the five types of regression equations calculated in the regression equation standard value calculation process (step 1102) . An AIC is calculated according to the following equation.

[Equation 1]

$$AIC = -2\left\{-\frac{n}{2}\left(\log(2\pi\sigma^2) + 1\right)\right\} + 2(P + 1)$$

In the equation for calculating the foregoing AIC, P is the number (number of functions of the regression equation) of parameters, and n is the number of data items. In the 0th order function, the number P of parameters is 1. In the linear function, the number P of parameters is 2. In the quadratic function, the number P of parameters is 3. In the logarithmic function, the number P of parameters is 4. In the exponential function, the number P of parameters is 4. The AIC calculated in step 1103 is used in a process (step 1105) of calculating the optimal regression equation.

(iv) Step 1104

The processor 3091 determines whether regression line standard values have been calculated for all the regression lines of the foregoing five types (example). When the calculation of all the regression line standard values is completed (YES in step 1104), the process proceeds to step 1105. When the calculation of all the regression line standard values is not yet completed (NO in step 1104), the process proceeds to step 1102, and the regression equation standard value calculation process (step 1102) and the AIC calculation process (step 1103) are repeated.

(v) Step 1105

The processor 3091 selects the optimal regression equation. For example, the processor 3091 compares AICs of the regression equations of all the five types and selects, as the optimal regression equation, a regression equation having the smallest AIC. As the number of parameters in a regression equation becomes larger, fitting to a reaction process data group becomes better. On the other hand, when fitting to reaction process data is too good, the accuracy of prediction for unknown data is reduced. When performance of regression using regression equations with different numbers of parameters is compared, an AIC is used as an index indicating the appropriateness of a regression equation for a data distribution in consideration of the number of parameters. As an AIC obtained from a regression equation becomes smaller, the regression equation becomes more appropriate for a data distribution.

In addition, the processor 3091 uses an evaluation parameter value of reaction process data targeted for deviation determination for the selected optimal regression equation to calculate a standard range of upper and lower limits of the integrated deviation determination standard line 904. The standard range of the upper and lower limits varies depending on the regression equation and is calculated as a coefficient for each of the 0th order function, the linear function, the quadratic function, the logarithmic function, and the exponential function. The coefficients are calculated as correlation functions using a combination of measurement data and each evaluation parameter 302. It is possible to determine whether the value is in the standard value range based on the calculated standard range of the upper and lower limits, and to use the data as reaction process data out of the range or deviation data.

<Conclusion>

(i) As described above, the automatic analyzer for clinical inspection measures, at certain time intervals, the absorbance of a solution in which a sample has reacted with a reagent, and measures the rate of change in the absorbance and the final absorbance based on the absorbance measured over time. The absorbance at the start time of the reaction is A0, the final absorbance in the reaction is A1, a reaction speed constant is k, the total of differences between approximate values and measured values is used as a residual error, and an evaluation parameter as an index for a reaction situation is calculated for each measurement item and each reagent lot for one automatic analyzer. The evaluation parameter is used as a distribution diagram of reference data and a regression line to determine an abnormality of the reaction process.

However, the distribution of the reference data and the regression line depend on a specimen. When results of the specimen are biased, the regression line is specialized for the automatic analyzer unit. It is considered that the accuracy of performing deviation determination using a regression line for one automatic analyzer is not sufficient to objectively determine a deviation in a reaction process for each of reagents used by a user and each of reagent lots due to a device difference specific to the device and the bias of the specimen.

To solve such a problem, the automatic-analysis data analysis system 300 according to the present embodiment has been devised.

(ii) In the automatic-analysis data analysis system 300 according to the present embodiment, the integrated analysis server 309 collects (acquires) reference data from a remote terminal accumulating the reference data (refer to Fig. 4) in units of automatic analysis systems of the same type, units of information (device lots) of the automatic analysis systems, units of automatic analysis systems that operate in all facilities in the same reagent unit or the same reagent lot unit, or units of automatic analysis systems connected. In addition, the integrated analysis server 309 uses the accumulated reference data to calculate integrated regression line information (information (device lot) of the same system and regression line information of entire reference data of the same reagent lot) and integrated deviation determination line information, and transmits the information to the automatic analysis systems that operate in all the facilities or the remote terminal accumulating the reference data. The remote terminal is not an essential configuration in the present embodiment. The integrated analysis server 309 may directly transmit the integrated regression line information and the integrated deviation determination line information to a computer of each automatic analysis system. The process of the integrated analysis server 309 can be executed by one remote terminal.

In addition, each automatic analyzer or each remote terminal displays, in a distribution diagram of reference data and a distribution diagram of measurement data, integrated regression line information, an integrated regression line calculated from a deviation determination standard line, and the deviation determination standard line.

Furthermore, each automatic analyzer or each remote terminal displays, on a screen of a display unit of the automatic analysis system or a screen of a display unit of the remote terminal, a regression line and a deviation determination standard line (a regression line and a deviation determination standard line of the single device) calculated from one automatic analysis system (one set), the integrated regression line, and the integrated deviation determination standard line simultaneously (in an overlapping manner).

The display unit is not limited to the foregoing and may be a display device included in at least any one system of a clinical inspection device, an inspection chamber information system, a hospital information system including an electronic medical record, which are operated by a healthcare professional.

The inspection chamber information system is a higher-level system of the clinical inspection device and controls the entire clinical inspection device. In addition, the hospital information system is a system for an electronic medical record or is an ordering system or the like and is for a terminal to be operated by a doctor. The hospital information system is a higher-level system of the inspection chamber information system.

(iii) According to the present embodiment, it is possible to present, to a user, a regression line and a deviation determination standard line that have reduced dependence on specimens and device differences. There has not been means for objectively determining a reaction curved line for each reagent and each reagent lot using only a regression line specific to an inspection chamber, but the effect of improving the accuracy of deviation determination by a user is obtained by adding an integrated index using regression lines acquired from all facilities.

In addition, when reaction process data deviates from a standard range of an integrated and single regression line, reinspection is performed. When the reaction process data does not deviate from a single regression line but deviates from an integrated regression line, a determination index for checking a reaction process in detail can be added and the effect of reducing a task of checking deviating data can be expected.

(iv) The present embodiment can be achieved by a program code of software that enables the functions described in the present embodiment. In this case, a storage medium storing the program code is provided to a system or a device, and a computer (or a CPU or an MPU) of the system or of the device reads the program code stored in the storage medium. In this case, the program code read from the storage medium enables the functions described in the foregoing embodiment, and the program code and the storage medium storing the program code constitute the present disclosure. As the storage medium for supplying the program code, for example, a flexible disk, a CD-ROM, a DVD-ROM, a hard disk, an optical disc, a magneto-optical disc, a CD-R, a magnetic table, a nonvolatile memory card, a ROM, or the like is used.

In addition, an OS (operating system) executed on the computer may execute a part of an actual process or the entire process based on an instruction of the program code, and the functions described above in the embodiment may be enabled by the process. Furthermore, after the program code read from the storage medium is written to a memory in the computer, the CPU or the like of the computer may execute a part of the actual process or the entire process in accordance with an instruction of the program code, and the functions described in the embodiment may be enabled by the process.

Furthermore, the software program code that enables the functions described in the present embodiment may be distributed via a network and stored in a storage unit such as hard disk or a memory of the system or of the device, or a storage medium such as a CD-RW or a CD-R, and the computer (or the CPU or the MPU) of the system or of the device may read and execute the program code stored in the storage unit or the storage medium at the time of the use.

Lastly, it is necessary to understand that the processes and techniques described here do not essentially relate to any specific device and can be implemented by any corresponding combination of components. Furthermore, various types of general-purpose devices can be used in accordance with the teaching described here. It may be found that it is beneficial to build a dedicated device to execute the steps of the method described here. In addition, various inventions can be formed by an appropriate combination of a plurality of constituent components disclosed in the embodiment. For example, some of all the constituent components described in the embodiment may be removed. Furthermore, constituent components described across different embodiments may be combined as appropriate. The present disclosure is described in connection with the specific examples, but these are not for limitation and are for explanation in all respects. Persons skilled in the art will find that there are many combinations of hardware, software, and firmware that are suitable to implement the present disclosure. For example, the described software can be implemented using a program or a script language in a wide range of assembly language, C/C++, perl, Shell, PHP, and Java (registered trademark).

Furthermore, in the foregoing embodiment, control lines and information lines that are considered to be necessary for the explanation are illustrated, and not all control and information lines are illustrated for the product. All the configurations may be connected to each other.

Description of Reference Signs

300 Automatic-analysis data analysis system
301, 304 Communication line (LAN)
302, 306, 307 Automatic analysis system
303, 310 Remote terminal
305, 308 Communication line (dedicated line or Internet line)
701 Reference data information table (reference data)
702 Evaluation parameter
703 Item code
704, 802 Device lot
705, 803 Reagent lot

**Claims**

1. A data analysis method comprising:

   causing each of a plurality of first computers to acquire measurement data obtained by allowing a sample to react with a reagent in one or more automatic analyzers; and
   causing a second computer connected to the plurality of first computers to analyze the measurement data acquired from each of the plurality of first computers and generate a result of the analysis, wherein
   in the generating of the result of the analysis, the second computer executes
   a process of acquiring reference data including information on the automatic analyzers, information on the reagent, and the measurement data associated with the information, and
   a process of generating integrated deviation determination line information commonly applicable to the plurality of automatic analyzers associated with the information on the automatic analyzers and the information on the reagent.

2. The data analysis method according to claim 1, wherein

   the second computer executes a process of transmitting the integrated deviation determination line information to the first computers, and
   the plurality of first computers execute a process of acquiring the integrated deviation determination line information transmitted from the second computer and displaying the integrated deviation determination line information on display screens of the first computers.

3. The data analysis method according to claim 2, wherein
   the first computers generate, from measurement data of a corresponding automatic analyzer, deviation determination line information specific to the automatic analyzer, and display, on the display screens, the integrated deviation determination line information generated by the second computer and the single deviation determination line information of the single automatic analyzer.

4. The data analysis method according to claim 3, wherein
   the first computers display, on the display screens, the integrated deviation determination line information and the single deviation determination line information of the single automatic analyzer in such a manner that the integrated deviation determination line information overlaps the single deviation determination line information of the single automatic analyzer.

5. The data analysis method according to claim 1, wherein
the second computer generates the integrated deviation determination line information from the reference data in units of the information on the automatic analyzers and the information on the reagent.

6. The data analysis method according to claim 2, wherein
the second computer executes a process of determining whether to transmit the generated integrated deviation determination line information before the transmitting process.

7. The data analysis method according to claim 6, wherein
the second computer determines whether to transmit the integrated deviation determination line information based on whether the automatic analyzers connected to the first computers use a reagent lot associated with information on the reagent.

8. The data analysis method according to claim 6, wherein
the second computer determines whether to transmit the integrated deviation determination line information based on whether an automatic analyzer associated with the information on the automatic analyzers is present among the automatic analyzers connected to the first computers.

9. A data analysis system comprising:

a plurality of first computers that acquire measurement data obtained by allowing a sample to react with a reagent in one or more automatic analyzers; and
a second computer that analyzes the measurement data acquired from each of the first computers and transmits a result of the analysis to the plurality of first computers, wherein
the second computer executes
a process of acquiring reference data including information on the plurality of automatic analyzers, information on the reagent, and the measurement data associated with the information, and
a process of generating, based on the reference data, integrated deviation determination line information commonly applicable to the plurality of automatic analyzers associated with the information on the automatic analyzers and the information on the reagent.

10. The data analysis system according to claim 9, wherein
the second computer executes a process of transmitting a plurality of integrated regression line information items and the integrated deviation determination line information to the first computers, and
the first computers execute a process of acquiring the integrated deviation determination line information transmitted from the second computer and displaying the integrated deviation determination line information on display screens of the first computers.

11. The data analysis system according to claim 10, wherein
the first computers generate, from measurement data of a corresponding automatic analyzer, deviation determination line information specific to the automatic analyzer and displays, on the display screens, the integrated deviation determination line information generated by the second computer and the single deviation determination line information of the single automatic analyzer.

12. The data analysis system according to claim 11, wherein
the first computers display, on the display screens, the integrated deviation determination line information and the single deviation determination line information of the single automatic analyzer in such a manner that the integrated deviation determination line information overlaps the single deviation determination line information of the single automatic analyzer.

13. The data analysis system according to claim 9, wherein
the second computer generates the integrated deviation determination line information from the reference data in units of the information on the automatic analyzers and the information on the reagent.

14. The data analysis system according to claim 10, wherein

the second computer executes a process of determining whether to transmit the generated integrated deviation determination line information before the transmitting process.

15. The data analysis system according to claim 14, wherein
the second computer determines whether to transmit the integrated deviation determination line information based on whether the automatic analyzers connected to the first computers use a reagent lot associated with the information on the reagent.

16. The data analysis system according to claim 14, wherein
the second computer determines whether to transmit the integrated deviation determination line information based on whether an automatic analyzer associated with the information on the automatic analyzers is present among the automatic analyzers connected to the first computers.

17. A computer comprising:

a storage unit that stores various information and various data;
a controller that reads the various information and/or the various data from the storage unit to execute information processing; and
a display device that outputs a result of the information processing to a display screen, wherein
the controller executes
a process of acquiring measurement data obtained by allowing a sample to react with a reagent in one or more automatic analyzers, and
a process of displaying an analysis result generated by analyzing the measurement data on the display screen of the display device, and
the analysis result includes integrated deviation determination line information generated based on reference data including information on the automatic analyzers, information on the reagent, and the measurement data associated with the information, and commonly applicable to the plurality of automatic analyzers associated with the information on the automatic analyzers and the information on the reagent.

18. The computer according to claim 17, wherein
the controller generates, from measurement data of a corresponding automatic analyzer, deviation determination line information specific to the automatic analyzer and displays, on the display screen, the integrated deviation determination line information and the single deviation determination line information of the single automatic analyzer.

# FIG. 1

# FIG. 2

# FIG. 3

# FIG. 4

309

| | |
|---|---|
| PROCESSOR 3091 | MEMORY ~3092 |
| 30911 | STORAGE DEVICE ~3093 |
| INTEGRATED REGRESSION LINE PROCESSING UNIT | INPUT DEVICE ~3094 |
| | OUTPUT DEVICE ~3095 |
| | COMMUNICATION DEVICE ~3096 |

# FIG. 5

303(310)

| | | |
|---|---|---|
| | MEMORY | ─3032 |
| | | |
| 3031 | STORAGE DEVICE | ─3033 |
| PROCESSOR | INPUT DEVICE | ─3034 |
| | OUTPUT DEVICE | ─3035 |
| | COMMUNICATION DEVICE | ─3036 |

# FIG. 6

# FIG. 7

| No | REFERENCE DATA | |
|----|----------------|---|
| | | ∼701 |
| 1 | APPROXIMATE EQUATION CODE | |
| 2 | ANALYSIS METHOD | |
| 3 | REQUEST TYPE | |
| 4 | SPECIMEN IDENTIFICATION CODE | |
| 5 | SPECIMEN TYPE | |
| 6 | ITEM NAME | |
| 7 | ITEM CODE | ∼703 |
| 8 | ANALYTICAL DISPENSING AMOUNT | |
| 9 | MEASURED VALUE | |
| 10 | DATA ALARM | |
| 11 | ANALYZING UNIT | |
| 12 | SYSTEM | |
| 13 | DEVICE LOT | ∼704 |
| 14 | REAGENT LOT | ∼705 |
| 15 | DISPENSING DATE AND TIME | |
| 16 | SUM Err OF SQUARES OF RESIDUAL ERRORS | |
| 17 | REACTION SPEED CONSTANT k | |
| 18 | AMOUNT A1 OF CHANGE IN ABSORBANCE | |
| 19 | ABSORBANCE A0 AT START TIME OF REACTION | 702 |
| 20 | INCLINATION p OF ASYMPTOTE | |
| 21 | INTERCEPT q OF ASYMPTOTE | |
| 22 | MAGNITUDE D0 OF LAG PHASE | |
| 23 | LENGTH T1 OF LAG PHASE | |
| 24 | ABSORBANCE (MAIN/AUXILIARY) 1 TO 38 | |
| 25 | APPROXIMATE VALUES 1 TO 38 | |

# FIG. 8

|  | | ITEM CODE (TP) | | |
|---|---|---|---|---|
| DEVICE LOT A | REAGENT LOT A | REFERENCE DATA 1 | SUM Err OF SQUARES OF RESIDUAL ERRORS | |
| | | | REACTION SPEED CONSTANT k | |
| | | | AMOUNT Al OF CHANGE IN ABSORBANCE | |
| | | | ABSORBANCE A0 AT START TIME OF REACTION | |
| | | | INCLINATION p OF ASYMPTOTE | |
| | | | INTERCEPT q OF ASYMPTOTE | |
| | | | MAGNITUDE D0 OF LAG PHASE | |
| | | | LENGTH T1 OF LAG PHASE | |
| | | | ABSORBANCE (MAIN/AUXILIARY) 1 TO 38 | |
| | | | APPROXIMATE VALUES 1 TO 38 | |
| | | REFERENCE DATA N | SUM Err OF SQUARES OF RESIDUAL ERRORS | |
| | | | REACTION SPEED CONSTANT k | |
| | | | AMOUNT A1 OF CHANGE IN ABSORBANCE | |
| | | | ABSORBANCE A0 AT START TIME OF REACTION | |
| | | | INCLINATION p OF ASYMPTOTE | |
| | | | INTERCEPT q OF ASYMPTOTE | |
| | | | MAGNITUDE D0 OF LAG PHASE | |
| | | | LENGTH T1 OF LAG PHASE | |
| | | | ABSORBANCE (MAIN/AUXILIARY) 1 TO 38 | |
| | | | APPROXIMATE VALUES 1 TO 38 | |
| | REAGENT LOT B | REFERENCE DATA N | ... | |
| DEVICE LOT B | REAGENT LOT A | REFERENCE DATA 1 | ... | |

23

# FIG. 9

# FIG. 10

START

COLLECT REFERENCE DATA — S1001

CHECK ITEM CODE — S1002
→ NOT PRESENT

PRESENT

CHECK DEVICE LOT — S1003
→ NOT PRESENT

PRESENT

CHECK REAGENT LOT — S1004
→ NOT PRESENT

PRESENT

REGISTER NEW ITEM CODE — S1006

CALCULATE INTEGRATED REGRESSION LINE — S1005

CHECK DISTRIBUTION DESTINATION — S1007
→ NOT PRESENT

PRESENT

DISTRIBUTE INTEGRATED REGRESSION LINE INFORMATION — S1009

NOTIFY ABNORMALITY — S1008

END

# FIG. 11

START

READ EVALUATION FACTOR — S1101

CALCULATE STANDARD
VALUE FOR REGRESSION
EQUATION — S1102

CALCULATE AIC — S1103

ALL
STANDARD LINE
TYPES HAVE BEEN
COMPLETED? — S1104

NO

YES

SELECT OPTIMAL
REGRESSION EQUATION — S1105

END

## INTERNATIONAL SEARCH REPORT

| International application No. |
|---|
| PCT/JP2020/010253 |

**A. CLASSIFICATION OF SUBJECT MATTER**
Int. Cl. G01N35/00(2006.01)i
FI: G01N35/00 F, G01N35/00 A

According to International Patent Classification (IPC) or to both national classification and IPC

**B. FIELDS SEARCHED**

Minimum documentation searched (classification system followed by classification symbols)
Int. Cl. G01N35/00

Documentation searched other than minimum documentation to the extent that such documents are included in the fields searched
Published examined utility model applications of Japan    1922-1996
Published unexamined utility model applications of Japan    1971-2020
Registered utility model specifications of Japan    1996-2020
Published registered utility model applications of Japan    1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 11-326332 A (HITACHI, LTD.) 26 November 1999, paragraphs [0010], [0024]-[0026], [0028]-[0033] | 1-18 |
| Y | JP 2003-4750 A (HITACHI, LTD.) 08 January 2003, paragraphs [0020], [0021], [0027], [0028], [0033], [0041] | 1-18 |
| Y | WO 2012/157386 A1 (HITACHI HIGH-TECHNOLOGIES CORP.) 22 November 2012, paragraphs [0024], [0031], [0036], [0046], [0053], [0055]-[0058], [0064], [0065], [0091]-[0096], [0135], fig. 16A | 1-18 |
| A | JP 2001-229291 A (SYSMEX CORP.) 24 August 2001, paragraph [0041] | 1-18 |

☒ Further documents are listed in the continuation of Box C.      ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 25.05.2020 | 09.06.2020 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**EP 3 951 398 A1**

<table>
<tr><td colspan="2" align="center">INTERNATIONAL SEARCH REPORT</td><td>International application No.<br>PCT/JP2020/010253</td></tr>
</table>

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2010/128575 A1 (HITACHI HIGH-TECHNOLOGIES CORP.) 11 November 2010, paragraphs [0035]-[0058] | 1-18 |

Form PCT/ISA/210 (continuation of second sheet) (January 2015)

**INTERNATIONAL SEARCH REPORT**

Information on patent family members

International application No.

PCT/JP2020/010253

| Patent Documents referred to in the Report | Publication Date | Patent Family | Publication Date |
|---|---|---|---|
| JP 11-326332 A | 26.11.1999 | (Family: none) | |
| JP 2003-4750 A | 08.01.2003 | (Family: none) | |
| WO 2012/157386 A1 | 22.11.2012 | US 2014/0136123 A1 paragraphs [0059], [0066], [0071], [0081], [0088], [0090]-[0093], [0099], [0100], [0126]-[0131], [0170], fig. 16A EP 002711713 A1 CN 103534596 A | |
| JP 2001-229291 A | 24.08.2001 | US 2002/0128801 A1 paragraph [0081], [0082] EP 001107159 A2 | |
| WO 2010/128575 A1 | 11.11.2010 | US 2012/0109534 A1 paragraphs [0044]-[0078] EP 002428802 A1 CN 102422162 A | |

Form PCT/ISA/210 (patent family annex) (January 2015)

**EP 3 951 398 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008047033 A

- JP 2014247715 A